# EUROPÄISCHE PATENTSCHRIFT

(11) **EP 1 250 316 B1**
(45) Veröffentlichungstag und Bekanntmachung des Hinweises auf die Patenterteilung: **29.12.2004**
(21) Anmeldenummer: 01913765.2
(22) Anmeldetag: 24.01.2001
(51) Int. Cl.: C07C 259/06, C07C 259/10, C07D 307/68

(54) **VERFAHREN ZUR HERSTELLUNG VON N-ALKOXY-N-ALKYLAMIDEN**
METHOD FOR PRODUCING N-ALKOXY-N-ALKYLAMIDES
PROCEDE DE PREPARATION DE N-ALCOXY-N-ALKYLAMIDES

(30) Priorität: 25.01.2000 EP 00101391; 12.05.2000 US 203906 P
(43) Veröffentlichungstag der Anmeldung: 23.10.2002
(73) Patentinhaber: Lonza AG, 4052 Basel (CH)
(72) Erfinder: HANSELMANN, Paul, CH-3902 Brig-Glis (CH); HILDBRAND, Stefan, CH-4125 Riehen (CH); STERREN, Etienne, CH-3902 Glis (CH)
(86) Internationale Anmeldenummer: PCT/EP2001/000753
(87) Internationale Veröffentlichungsnummer: WO 2001/055096

(56) Entgegenhaltungen:
- WO-A-00/32564
- GB-A- 852 176
- J. MICHAEL WILLIAMS ET AL.: "A New General Method for Preparation of N-Methoxy-N-Methylamides. Application in Direct Conversion of an Ester to a Ketone" TETRAHEDRON LETTERS, Bd. 36, Nr. 31, 31. Juli 1995 (1995-07-31), Seiten 5461-5464, XP002168325 OXFORD GB
- TAKESHI SHIMIZU ET AL.: "Efficient Method for Preparation of N-Methoxy-N-methyl Amides by Reaction of Lactones or Esters with Me2AlCl-MeONHMe.HCl " TETRAHEDRON LETTERS, Bd. 38, Nr. 15, 1997, Seiten 2685-2688, XP004058304 OXFORD GB

## Beschreibung

Die Erfindung betrifft ein Verfahren zur Herstellung von *N*-Alkoxy-*N*-alkylamiden.

Unter den *N*-Alkoxy-*N*-alkylamiden besitzen die *N*-Methoxy-*N*-methylamide ("Weinreb-Amide") die weitaus grösste Bedeutung. Die Chemie der Weinreb-Amide wurde in mehreren Übersichtsartikeln zusammengefasst; so zum Beispiel von M. Mentzel, H. M. R. Hoffmann, *J*. *prakt*. *Chem*. **1997,** *339*, 517-524 oder M. P. Sibi, *Org. Preparations and Procedures Int.* **1993,** *25(1),* 15-40.

Die bekannten Verfahren zur Herstellung von *N*-Methoxy-*N*-methylamiden haben den Nachteil, dass praktisch ausnahmslos *N*,*O*-Dimethylhydroxylamin als Reagens eingesetzt wird. Da dieses Reagens vergleichsweise teuer ist, ist eine technische Anwendung von Weinreb-Amiden limitiert.

Aufgabe der vorliegenden Erfindung ist daher, ein alternatives und kostengünstigeres Verfahren zur Herstellung von *N*-Alkoxy-*N*-alkylamiden und insbesondere von *N*-Methoxy-*N*-methylamiden bereitzustellen.

Erfindungsgemäss wird diese Aufgabe durch das Verfahren nach Patentanspruch 1 gelöst.

Es wurde gefunden, dass *N*-Alkoxy-*N*-alkylamide der allgemeinen Formel I 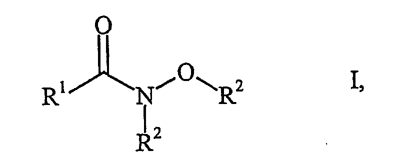 worin
- R¹: C₁₋₁₀-Alkyl, Cyclo-C₅₋₇-Alkyl, Cyclo-C₅₋₇-alkenyl, C₂₋₁₀-Alkenyl, Aryl, Aryl-C₁₋₃-alkyl, Heteroaryl, Heteroaryl-C₁₋₃-alkyl oder Heterocyclyl; und
- R²: C₁₋₆-Alkyl bedeuten;
dadurch hergestellt werden können, dass ein Ester der allgemeinen Formel

R¹COOR³ II,

worin R¹ die oben angegebene Bedeutung hat und R³ C₁₋₆-Alkyl, 4-Nitrophenyl, 2,4-Dinitrophenyl, Succinimido (2,5-Dioxopyrrolidin-1-yl) oder Benzotriazol-1-yl bedeutet, mit Hydroxylamin, einem Hydroxylaminderivat oder einem Hydroxylammoniumsalz umgesetzt wird und das Reaktionsprodukt in Gegenwart eines Phasentransferkatalysators alkyliert wird.

Unter C₁₋₁₀-Alkyl sind hier und im folgenden alle linearen oder verzweigten Alkylgruppen mit 1-10 Kohlenstoffatomen zu verstehen wie beispielsweise Methyl, Ethyl, Propyl, Isopropyl, Butyl, Isobutyl, *sec*-Butyl, *tert*-Butyl, Pentyl, Isopentyl, *tert*-Pentyl, Neopentyl, Hexyl, Isohexyl, Octyl, Nonyl oder Decyl.

Unter Cyclo-C₅₋₇-alkyl sind ringförmige Kohlenwasserstoffreste mit 5-7 Kohlenstoffatomen zu verstehen wie beispielsweise Cyclopentyl, Cyclohexyl oder Cycloheptyl.

Unter Cyclo-C₅₋₇-alkenyl sind ringförmige Kohlenwasserstoffreste mit 5-7 Kohlenstoffatomen zu verstehen, wobei der Ring eine Doppelbindung enthält, wie beispielsweise Cyclopentenyl oder Cyclohexenyl.

Unter C₂₋₁₀-Alkenyl sind lineare oder verzweigte Alkenylgruppen mit 2-10 Kohlenstoffatomen zu verstehen, wie beispielsweise Vinyl, Allyl, Methallyl, die Reste Butenyl, Pentenyl, Hexenyl, Heptenyl, Octenyl sowie deren Isomere, 2-Methyl-1-propenyl, 2-Methyl-1-butenyl, 1-Methyl-2-butenyl, 2-Methyl-2-butenyl, 3-Methyl-2-butenyl, 2,3-Dimethyl-2-butenyl, 1,3-Dimethyl-2-butenyl, 1-Ethyl-2-butenyl, 4-Methyl-2-pentenyl, 2-Ethyl-2-pentenyl, 4,4-Dimethyl-2-pentenyl oder 1,4-Dimethyl-1-hexenyl.

Unter Aryl sind aromatische Kohlenwasserstoffreste zu verstehen wie beispielsweise Phenyl, 1-Naphthyl, 2-Naphthyl, 1-Anthryl, 2-Anthryl, 9-Anthryl oder Biphenylyl, vorzugsweise Phenyl. Die Arylgruppen können auch einen oder mehrere gleiche oder verschiedene Substituenten wie C₁₋₆-Alkyl, C₁₋₆-Alkoxy oder Halogen in ortho-, meta- oder para-Stellung tragen. Geeignete substituierte Aryl-Reste sind beispielsweise Methylphenyl, Dimethylphenyl, Ethylphenyl, Propylphenyl, Methoxyphenyl, Ethoxyphenyl, Propoxyphenyl, Methylnaphthyl oder Methoxynaphthyl.

Unter Aryl-C₁₋₃-alkyl sind beispielsweise Reste wie Benzyl, 1-Phenylethyl, 2-Phenylethyl, 3-Phenylpropyl, 1-Naphthylmethyl oder 2-Naphthylmethyl zu verstehen. Bevorzugt ist Benzyl. Die Arylgruppen können auch wie oben erwähnt einen oder mehrere gleiche oder verschiedene Substituenten in ortho-, meta- oder para-Stellung tragen. Geeignete substituierte Aryl-C₁₋₃-alkyl Reste sind beispielsweise Methylbenzyl, Methoxybenzyl, Methylphenylethyl oder Methylnaphthylmethyl.

Unter Heteroaryl sind Reste wie beispielsweise Pyridyl, Pyrazinyl, Pyrimidinyl, Pyridazinyl, Chinolinyl, Isochinolinyl, Pyrrolyl, Pyrazolyl, Imidazolyl, Benzimidazolyl, Thiazolyl, Oxazolyl, Thiophenyl, Benzothiophenyl, Furanyl, Benzofuranyl oder Indolyl zu verstehen.

Die oben aufgeführten Heteroarylreste können ebenfalls durch Methyl-, Ethyl-, oder Propylreste sowie durch Methoxy-, Ethoxy-, Propoxyreste substituiert sein.

Unter Heteroaryl-C₁₋₃-alkyl sind Methyl-, Ethyl-, oder Propylreste zu verstehen, die durch die oben genannten Heteroarylreste substituiert sind, wie beispielsweise Pyridylmethyl, Pyrazinylethyl, Pyrimidylpropyl und dergleichen.

Unter Heterocyclyl sind nicht-aromatische heterocyclische Reste wie beispielsweise Morpholinyl, Tetrahydrofuranyl, Dihydrofuranyl, Tetrahydrothiophenyl, Dihydrothiophenyl, Pyrrolidinyl, Imidazolinyl, Pyrazolinyl, Piperidinyl, Piperazinyl und dergleichen zu verstehen.

Geeignete Phasentransferkatalysatoren umfassen quartäre Ammonium- oder Phosphoniumsalze oder tertiäre Amine. Bevorzugt sind quartäre Ammoniumsalze wie beispielsweise Tetra-*n*-C₁₋₁₀-alkylammonium-, Benzyltri-*n*-C₁₋₁₀-alkylammonium- und Methyltri-*n*-C₄₋₁₀-alkylammoniumhalogenide, wobei Halogenid vorzugsweise für Chlorid oder Bromid steht beim Vorhandensein und mehrerer Alkylgruppen diese gleiche oder verschiedene Kettenlängen aufweisen können. Besonders bevorzugt ist Tetrabutylammoniumbromid.
Die genannten Phasentransferkatalysatoren sind im Handel erhältlich oder nach bekannten Verfahren herstellbar.

Unter Hydroxylaminderivaten sind beispielsweise Verbindungen der Formel H₂N-OR^{a} zu verstehen, wobei R^{a} C₁₋₃-Alkyl bedeutet, beispielsweise *O*-Methylhydroxylamin.

Beispiele von Hydroxylammoniumsalzen sind Hydroxylammonium-sulfat und Hydroxylammonium-chlorid. Beide Produkte sind im Handel erhältich.

Erfindungsgemäss einsetzbare Alkylierungsmittel sind C₁₋₆-Alkylhalogenide, vorzugsweise Alkylchloride oder Alkylbromide. Besonders bevorzugt ist Methylchlorid.

Die Ester der Formel R¹COOR³ sind im Handel erhältlich oder können durch bekannte Veresterungsverfahren hergestellt werden, z. B. durch Umsetzung der entsprechenden Säure R¹COOH mit einem C₁₋₆-Alkanol, 4-Nitrophenol, 2,4-Dinitrophenol, *N*-Hydroxysuccinimid oder 1-Hydroxybenzotriazol.

Das erfindungsgemässe Verfahren wird vorteilhafterweise als "Eintopfverfahren" durchgeführt, wobei zunächst ein Ester mit Hydroxylamin, einem Hydroxylaminderivat oder einem Hydroxylammoniumsalz in Gegenwart einer Base bei einer Temperatur von -20 bis 100 °C umgesetzt und das Reaktionsprodukt in Gegenwart eines Phasentransferkatalysators bei einer Temperatur von 10 bis 120 °C und einem Druck von 1 bis 50 bar alkyliert wird. Das alkylierte Produkt wird nach bekannten Methoden isoliert und aufgearbeitet, beispielsweise durch Extraktion.

Als Base wird vorzugsweise ein Alkalihydroxid oder Alkalicarbonat eingesetzt. Besonders bevorzugt ist Natriumhydroxid.

Die Umsetzung des Esters R¹COOR³ mit Hydroxylamin, einem Hydroxylaminderivat oder einem Hydroxylammoniumsalz wird in einem geeigneten Lösungsmittel durchgeführt.

Geeignete Lösungsmittel sind Wasser, Alkohole, wie beispielsweise Methanol, Ethanol, Propanol und Isopropanol oder Ether wie beispielsweise Tetrahydrofuran oder Dioxan. Auch Mischungen der genannten Lösungsmittel können eingesetzt werden. Ein bevorzugtes Lösungsmittel ist Wasser.

Hydroxylamin wird in äquimolarer Menge oder im geringen Überschuss (bezogen auf den Ester) eingesetzt.

Die folgenden Beispiele verdeutlichen die Durchführung des erfindungsgemässen Verfahrens, ohne dass darin eine Einschränkung zu sehen ist.

### Beispiel 1

### N-Methoxy-N-methyl-2-furancarboxamid

Zu einer Lösung von 4,18 g (0,025mol) Hydroxylammoniumsulfat und 5,66 g (0,045 mol) Furan-2-carbonsäuremethylester in 20 ml H₂O wurde unter kräftigem Rühren eine Lösung von 4,22 g (0,11 mol) NaOH in 15 ml H₂O während 15 Minuten so zugegeben, dass die Temperatur 30 °C nicht überstieg. Nach 2 Stunden Rühren bei 40 °C wurde das bei der Reaktion entstandene Methanol vollständig abdestilliert. Der Rückstand wurde in einen Druckautoklaven transferiert und 9,65 g (0,09 mol) Na₂CO₃, gefolgt von 2,99 g Tetrabutylammoniumbromid, wurden zugegeben. Anschliessend wurde der Autoklav geschlossen und 22,7 g (0,45 mol) Methylchlorid wurden aufgepresst. Nach 15 Stunden bei 40 °C (Ölbadtemperatur) wurde der Autoklav entspannt. Der pH-Wert wurde mit 1 M HCl auf 5 eingestellt und die wässrige Phase mit Ethylacetat (4×100 ml) extrahiert. Die organische Phase wurde getrocknet (Na₂SO₄) und das Lösungsmittel am Rotationverdampfer abdestilliert. Reinigung des Rückstandes mittels Flash-Säulenchromatographie (Kieselgel; Hexan/Ethylacetat 3:1) lieferte 4,16 g (60%) des *N*-Methoxy-*N*-methylamids als gelboranges Öl.
- ¹H NMR (400 MHz,CDCl₃):: 7,60 (m, 1H); 7,15 (m, 1H); 6,53 (m, 1M); 3,75 (s, 3H); 3,34 (s, 3H).
- ¹³C NMR (400 MHz,CDCl₃):: 159,20 (C=O); 145,81 (C); 145,23 (CH); 117,36 (CH); 111,60 (CH); 61,38 (OCH₃); 33,20 (NCH₃).

### Beispiel 2

### N-Methoxy-N-methyl-methacrylamid

Zu einer auf 0 °C abgekühlten Lösung von 4,19 g (0,026 mol) Hydroxylammoniumsulfat und 4,51 g (0,045 mol) Methacrylsäuremethylester in 20 ml H₂O wurde eine Lösung von 4,0 g (0,10 mol) NaOH in 15 ml H₂O während 25 Minuten so zugegeben, dass die Temperatur 4 °C nicht überstieg. Nach 2 Stunden bei 0 °C wurde das Reaktionsgemisch in einen Druckautoklaven transferiert und 9,56 g (0,09 mol) Na₂CO₃, gefolgt von 2,99 g Tetrabutylammoniumbromid, wurden zugegeben. Anschliessend wurde der Autoklav geschlossen und 23,7 g (0,47 mol) Methylchlorid wurden aufgepresst. Nach 15 Stunden bei 40 °C (Ölbadtemperatur) wurde der Autoklav entspannt. Der pH-Wert wurde mit 1 M HCl auf 5 eingestellt und die wässrige Phase mit Ethylacetat (4×100 ml) extrahiert. Die organische Phase wurde getrocknet (Na₂SO₄) und das Lösungsmittel am Rotationverdampfer vorsichtig abdestilliert. Reinigung des Rückstandes (5,03 g) mittels Flash-Säulenchromatographie (Silicagel; Hexan/Ethylacetat 4:1) lieferte 2.96 g (51%) des *N*-Methoxy-*N*-methylamids als leicht gelbliches Öl.
- ¹H NMR (400 MHz,CDCl₃):: 5,3 (s, 1H); 5,25 (s, 1H); 3,65 (s, 3H); 3,25 (s, 3H); 2,0 (s, 3H).
- ¹³C NMR (400 MHz,CDCl₃):: 171,61 (C=O); 140,27 (C); 117,41 (CH₂); 61,24 (OCH₃); 33,37 (NCH₃); 19,90 (CH₃).

### Beispiel 3

### N-Methoxy-N-methyl-benzamid

Zu einer Lösung von 4,21 g (0,025 mol) Hydroxylammoniumsulfat und 6,18 g (0,045 mol) Benzoesäuremethylester in 20 ml H₂O wurde bei 25 °C eine Lösung von 4,14 g (0,10 mol) NaOH in 15 ml H₂O während 30 Minuten zugegeben. Das resultierende Gemisch wurde nach 2 Stunden Rühren bei 40 °C in einen Autoklaven übergeführt. 4,50 g (0,11 mol) NaOH und 3,13 g Tetrabutylammoniumbromid wurden zugegeben und anschliessend 22,7 g (0,45 mol) MeCl aufgepresst. Nach 3 Stunden bei 100 °C (Ölbadtemperatur) wurde der Autoklav entspannt. Das Gemisch wurde mit 1 M HCl auf einen pH-Wert von 5 gestellt und mit Ethylacetat (4×100ml) extrahiert. Die organische Phase wurde getrocknet (Na₂SO₄) und das Lösungsmittel am Rotationverdampfer abdestilliert. Reinigung des Rückstandes mittels Kugelrohr-Destillation lieferte 3,42 g (46%) des *N*-Methoxy-*N*-methylamids als farbloses Öl.
- ¹H NMR (400 MHz, CDCl₃):: 7,65-7,70 (m, 2H); 7,36-7,48 (m, 3H); 3,55 (s, 3H); 3,35 (s, 3H).
- ¹³C NMR (400 MHz, CDCl₃):: 169,99 (C=O); 134,22 (C); 130,55 (CH); 128,16 (CH); 128,01 (CH); 61,02 (OCH₃); 33,80 (NCH₃).

### Beispiel 4

### N-Methoxy-N-methyl-2-phenylacetamid

Zu einer Lösung von 4,11 g (0,025 mol) Hydroxylammoniumsulfat und 6,78 g (0,045 mol) Phenylessigsäuremethylester in 20 ml H₂O wurde bei 25 °C eine Lösung von 4,16 g (0,104 mol) NaOH in 15 ml H₂O während 30 Minuten zugetropft. Das leicht gelbliche Reaktionsgemisch wurde 2 Stunden bei 40 °C gerührt und anschliessend in einen Autoklaven übergeführt. Nach Zugabe von 3,87 g (0,097 mol) NaOH und 3,13 g Tetrabutylammoniumbromid wurde der Autoklav geschlossen und 22,70 g (0,45 mol) Methylchlorid wurden aufgepresst. Nach 3 Stunden bei 60 °C (Ölbadtemperatur) wurde der Autoklav entspannt, der pH-Wert wurde mit 1 m NaOH auf 14 eingestellt und das Gemisch mit Ethylacetat (4×100ml) extrahiert. Die organische Phase wurde getrocknet (Na₂SO₄) und das Lösungsmittel am Rotationverdampfer abdestilliert. Reinigung des Rückstands (6,28 g) mittels Flash-Säulenchromatographie (Silicagel; Hexan/Ethylacetat 3:1) lieferte 4,21 g (52%) *N*-Methoxy-*N*-methyl-2-phenyl-acetamid als leicht gelbliches Öl.
- ¹H NMR (400 MHz, CDCl₃):: 7,20-7,36 (m, 5H); 3,76 (s, 2H); 3,58 (s, 3H); 3,18 (s, 3H).
- ¹³C NMR (400 MHz,CDCl₃):: 172,43 (C=O); 135,04 (C); 129,33 (CH); 128,52 (CH); 126,79 (CH); 61,27 (OCH₃); 39,44 (CH₂); 32,29 (NCH₃).

### Beispiel 5

### N-Methoxy-N,3-dimethyl-2-butenamid

Zu einer auf 0 °C abgekühlten Lösung von 4,23 g (0,026 mol) Hydroxylammoniumsulfat und 5,16 g (0,045 mol) 3.3-Dimethylacrylsäuremethylester in 20 ml H₂O wurde eine Lösung von 4,29 g (0,11 mol) NaOH in 15 ml H₂O während 20 Minuten so zugegeben, dass die Temperatur 4 °C nicht überstieg. Nach 6 Stunden bei 0 °C wurde das Reaktionsgemisch in einen Druckautoklaven übergeführt und 3,54 g (0,09 mol) NaOH, gefolgt von 3,54 g Tetrabutylammoniumbromid, wurden zugegeben. Anschliessend wurde der Autoklav geschlossen und 22,7 g (0,45 mol) Methylchlorid wurden aufgepresst. Nach 15 Stunden bei 45 °C (Ölbadtemperatur) wurde der Autoklav entspannt. Das Reaktionsgemisch (der pH betrug 14) wurde mit Diethylether (4×100ml) extrahiert. Die organische Phase wurde getrocknet (Na₂SO₄) und das Lösungsmittel am Rotationsverdampfer vorsichtig abdestilliert. Es wurden 2,58 g (40%) des *N*-Methoxy-*N*-methylamids als leicht gelbliches Öl erhalten.
- ¹H NMR (400 MHz, CDCl₃):: 6,13 (s, 1H); 3,67(s, 3H); 3,20 (s, 3H); 2,14 (s, 3H); 1,90 (s, 3H).
- ¹³C NMR (400 MHz,CDCl₃):: 168,16 (C=O); 153,03 (C); 114,44 (CH); 61,41 (OCH₃); 32,29 (NCH₃); 27,61 (CH₃); 20,19 (CH₃).

## Patentansprüche

1. Verfahren zur Herstellung von *N*-Alkoxy-*N*-alkylamiden der allgemeinen Formel 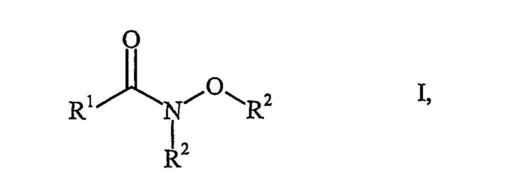 worin
R¹ C₁₋₁₀-Alkyl, Cyclo-C₅₋₇-alkyl, Cyclo-C₅₋₇-alkenyl, C₂₋₁₀-Alkenyl, Aryl, Aryl-C₁₋₃-alkyl, Heteroaryl, Heteroaryl-C₁₋₃-alkyl oder Heterocyclyl und
R² C₁₋₆-Alkyl bedeuten;
**dadurch gekennzeichnet, dass** ein Ester der allgemeinen Formel
R¹COOR³ II,
worin R¹ die oben angegebene Bedeutung hat und R³ C₁₋₆-Alkyl, 4-Nitrophenyl, 2,4-Dinitrophenyl, Succinimido oder Benzotriazol-1-yl bedeutet, mit Hydroxylamin, einem Hydroxylaminderivat oder einem Hydroxylammoniumsalz umgesetzt wird und das Reaktionsprodukt in Gegenwart eines Phasentransferkatalysators alkyliert wird.

2. Verfahren gemäss Anspruch 1, **dadurch gekennzeichnet, dass** R² Methyl bedeutet.

3. Verfahren gemäss Anspruch 1 oder 2, **dadurch gekennzeichnet, dass** R¹ C₂₋₁₀-Alkenyl, Aryl, Aryl-C₁₋₃-alkyl oder Heteroaryl bedeutet.

4. Verfahren gemäss Anspruch 3, **dadurch gekennzeichnet, dass** R¹ Phenyl, Benzyl, Furanyl, Methallyl oder 2-Methyl-1-propenyl bedeutet.

5. Verfahren gemäss einem der Ansprüche 1 bis 4, **dadurch gekennzeichnet, dass** der Phasentransferkatalysator ein quartäres Ammonium- oder Phosphoniumsalz oder ein tertiäres Amin ist.

6. Verfahren gemäss Anspruch 5, **dadurch gekennzeichnet, dass** der Phasentransferkatalysator Tetrabutylammoniumbromid ist.

7. Verfahren gemäss einem der Ansprüche 1 bis 6, **dadurch gekennzeichnet, dass** die Alkylierung mittels einer Verbindung der Formel
R²-X III
erfolgt, worin R² die in Anspruch 1 angegebene Bedeutung hat und X ein Halogenatom bedeutet.

8. Verfahren gemäss Anspruch 7, **dadurch gekennzeichnet, dass** als Hydroxylammoniumsalz Hydroxylammoniumsulfat eingesetzt wird und das Reaktionsprodukt in Gegenwart von Tetrabutylammoniumbromid mit Methylchlorid alkyliert wird.

## Claims

1. Process for the preparation of *N*-alkoxy-*N*-alkylamides of the general formula 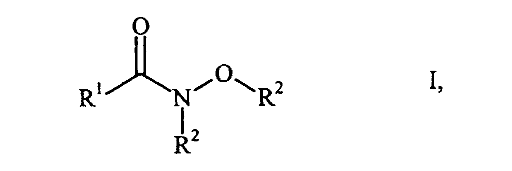 in which
R¹ is C₁₋₁₀ alkyl, C₅₋₇ cycloalkyl, C₅₋₇ cycloalkenyl, C₂₋₁₀ alkenyl, aryl, aryl-C₁₋₃ alkyl, heteroaryl, heteroaryl-C₁₋₃ alkyl or heterocyclyl; and
R² is C₁₋₆ alkyl;
**characterized in that** an ester of the general formula
R¹COOR³ II,
in which R¹ has the meaning indicated above and R³ is C₁₋₆ alkyl, 4-nitrophenyl, 2,4-di-nitrophenyl, *N*-succinimido or benzotriazol-1-yl, is reacted with hydroxylamine, a hydroxylamine derivative or a hydroxylammonium salt and the reaction product is alkylated in the presence of a phase-transfer catalyst.

2. Process according to Claim 1, **characterized in that** R² is methyl.

3. Process according to Claim 1 or 2, **characterized in that** R¹ is C₂₋₁₀ alkenyl, aryl, aryl-C₁₋₃ alkyl or heteroaryl.

4. Process according to Claim 3, **characterized in that** R¹ is phenyl, benzyl, furanyl, methallyl or 2-methyl-1-propenyl.

5. Process according to one of Claims 1 to 4, **characterized in that** the phase-transfer catalyst is a quaternary ammonium or phosphonium salt or a tertiary amine.

6. Process according to Claim 5, **characterized in that** the phase-transfer catalyst is tetrabutylammonium bromide.

7. Process according to one of Claims 1 to 6, **characterized in that** the alkylation is carried out by means of a compound of the formula
R²-X III
in which R² has the meaning indicated in Claim 1 and X is a halogen atom.

8. Process according to Claim 7, **characterized in that** hydroxylammonium sulfate is employed as hydroxylammonium salt and the reaction product is alkylated with methyl chloride in the presence of tetrabutylammonium bromide.

## Revendications

1. Procédé pour la préparation de *N*-alcoxy-*N*-alkylamides de formule générale 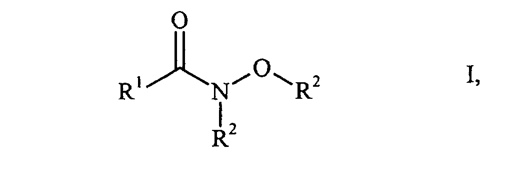 dans laquelle
R¹ représente un groupe alkyle en C₁-C₁₀, cycloalkyle en C₅-C₇, cycloalcényle en C₅-C₇, alcényle en C₂-C₁₀, aryle, aryl-alkyle(C₁-C₃), hétéroaryle, hétéroaryl-alkyle(C₁-C₃) ou hétérocyclyle et
R² représente un groupe alkyle en C₁-C₆ ;
**caractérisé en ce qu'**on fait réagir un ester de formule générale
R¹COOR³ II,
dans laquelle R¹ a la signification indiquée ci-dessus et R³ représente un groupe alkyle en C₁-C₆, 4-nitrophényle, 2,4-dinitrophényle, succinimido ou benzotriazol-1-yle, avec de l'hydroxylamine, un dérivé d'hydroxylamine ou un sel d'hydroxylammonium, et le produit de réaction est alkylé en présence d'un catalyseur de transfert de phase.

2. Procédé selon la revendication 1, **caractérisé en ce que** R² représente le groupe méthyle.

3. Procédé selon la revendication 1 ou 2, **caractérisé en ce que** R¹ représente un groupe alcényle en C₂-C₁₀) aryle, aryl-alkyle(C₁-C₃) ou hétéroaryle.

4. Procédé selon la revendication 3, **caractérisé en ce que** R¹ représente le groupe phényle, benzyle, furannyle, méthallyle ou 2-méthyl-1-propényle.

5. Procédé selon l'une quelconque des revendications 1 à 4, **caractérisé en ce que** le catalyseur de transfert de phase est un sel d'ammonium ou de phosphonium quaternaire ou une amine tertiaire.

6. Procédé selon la revendication 5, **caractérisé en ce que** le catalyseur de transfert de phase est le bromure de tétra-butylammonium.

7. Procédé selon l'une quelconque des revendications 1 à 6, **caractérisé en ce que** l'alkylation s'effectue au moyen d'un composé de formule
R²-X III
dans laquelle R² a la signification indiquée dans la revendication 1 et X représente un atome d'halogène.

8. Procédé selon la revendication 7, **caractérisé en ce qu'**on utilise en tant que sel d'hydroxylammonium du sulfate d'hydroxylammonium et le produit de réaction est alkylé avec du chlorure de méthylène en présence de bromure de tétra-butylammonium.
